# EUROPÄISCHE PATENTSCHRIFT

(11) **EP 0 652 799 B1**
(45) Veröffentlichungstag und Bekanntmachung des Hinweises auf die Patenterteilung: **20.11.1996**
(21) Anmeldenummer: 93915971.1
(22) Anmeldetag: 21.07.1993
(51) Int. Cl.: B01D 19/04

(54) **ENDGRUPPENVERSCHLOSSENE ANTISCHAUMMITTEL**
TERMINAL-GROUP-CLOSED ANTI-FOAMING AGENTS
AGENTS ANTIMOUSSE FERMES AU NIVEAU DU GROUPE TERMINAL

(30) Priorität: 30.07.1992 DE 4225236
(43) Veröffentlichungstag der Anmeldung: 17.05.1995
(73) Patentinhaber: Henkel Kommanditgesellschaft auf Aktien, 40191 Düsseldorf (DE)
(72) Erfinder: WANGEMANN, Frank, D-42697 Solingen (DE); RATHS, Hans-Christian, D-40789 Monheim (DE); POLY, Wolfgang, D-31079 Eberholzen (DE); GRUBER, Bert, D-50181 Bedburg (DE); GEKE, Jürgen, D-40225 Düsseldorf (DE); STEDRY, Bernd, D-47906 Kempen (DE)
(74) Vertreter: Wilk, Hans-Christof, Dr.
(86) Internationale Anmeldenummer: EP9301938
(87) Internationale Veröffentlichungsnummer: WO9403251

(56) Entgegenhaltungen:
- EP-A- 0 324 340
- WO-A-91/00763
- WO-A-92/11073
- Beilsteins Handbuch der organischen Chemie, 4. Aufl., drittes Ergänzungswerk, zweiter Band, erster Teil, Springer Verlag, 1960, Seiten 304-8

## Beschreibung

### Gebiet der Erfindung

Die Erfindung betrifft die Verwendung neuer endgruppenverschlossener Antischaummittel, die man durch vollständige oder teilweise Veresterung von Fettalkoholpolyglycolethern mit Carbonsäuren erhält, als Entschäumer in einer Vielzahl von Anwendungen.

### Stand der Technik

Für eine Vielzahl technischer Prozesse stellt die mit dem Einsatz von grenzflächenaktiven Substanzen verbundene Schaumentwicklung ein ernsthaftes Problem dar. Bei der maschinellen Haushaltsreinigung von Geschirr, aber auch der großtechnischen Reinigung von Bier- und Milchflaschen werden beispielsweise pulverförmige oder flüssige alkalische Reinigungsmittel verwendet, die neben Tensiden, Gerüstsubstanzen, Alkalimetallsilicaten, -carbonaten und/oder -hydroxiden zusätzlich Aktivchlor- oder Aktivsauerstoffverbindungen enthalten. Um ein übermäßiges Schäumen zu verhindern, wird diesen Mitteln in der Regel ein Antischaummittel zugesetzt.

Auch bei der Herstellung von Farben und Lacken, beispielsweise Wasserlacken, beobachtet man die Neigung der wasserverdünnbaren Bindemittel zu verstärkter Schaumbildung. Schaum kann hier bereits bei der Vermischung der Rezepturbestandteile entstehen, d. h. beim Anreiben des Lackes durch Einrühren von Luft, und führt dazu, daß die jeweiligen Anlagen nur mit einem Bruchteil des zu Verfügung stehenden Volumens gefüllt und betrieben werden können. Auch in diesem Bereich ist die Verwendung von Entschäumern und Entlüftern dringend geboten [**Goldschmidt. Inform. 66, 9 (1989)**].

Ein weiteres Feld, in dem Antischaummittel Einsatz finden, ist die Abwasserbehandlung von Kläranlagen. Beim Belebungsverfahren wird beispielsweise in das Abwasser Luft eingeblasen, wodurch eine innige Durchmischung und das Zusammentreffen der Schmutzteilchen mit den Mikroorganismen sichergestellt wird [**ChiuZ 25, 87 (1991)**]. Der hierbei entstehende Schaum ist in vielen Fällen Eiweißschaum und daher besonders beständig. Anstelle einer Sedimentation findet eine unerwünschte Fixierung der Feststoffe in und an den Schaumlamellen statt, was eine Abtrennung des Klärschlamms erschwert.

In der Vergangenheit hat es nicht an Versuchen gefehlt, für das komplexe Gebiet der Schaumregulierung entsprechende Antischaummittel zur Verfügung zu stellen.

Neben den wohlbekannten Schaumregulatoren, wie beispielsweise langkettigen Seifen, Copolymeren des Ethylen- und Propylenoxids oder Siliconen, haben sich in den letzten Jahren endgruppenverschlossene Fettalkoholpolyglycolether bewährt. Diese sogenannten "Mischether" werden im Sinne einer Williamson'schen Ethersynthese durch Umsetzung von Fettalkoholpolyglycolethern mit Alkylhalogeniden erhalten [**DE-C1-37 44 525**, Henkel]. Die Herstellung dieser Stoffe ist jedoch mit zwei Nachteilen behaftet: zum einen findet die Reaktion in Gegenwart stöchiometrischer Mengen eines Alkalihydroxids statt und ist dementsprechend mit einem hohen Salzanfall verbunden, zum anderen erfordert das Arbeiten mit Alkylhalogeniden hohe Anforderungen an den Arbeitsschutz.

Aus der Deutschen Offenlegungsschrift **DE-A1-40 41 184** (BASF) sind ferner Ester von Fettalkoholen mit Di- und Polycarbonsäuren bekannt, die als Entschäumer wirken. Für viele Anwendungen sind diese Produkte jedoch nicht ausreichend wasserlöslich.

Die Aufgabe der Erfindung bestand somit darin, neue Antischaummittel zur Verfügung zu stellen, deren Herstellung frei von den geschilderten Nachteilen ist.

### Beschreibung der Erfindung

Gegenstand der Erfindung ist die Verwendung von endgruppenverschlossenen Antischaummitteln, die man erhält, indem man Fettalkoholpolyglycolether der Formel (I) 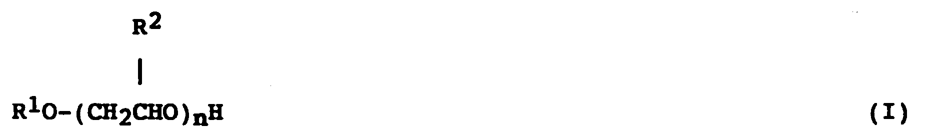 in der R¹ für einen aliphatischen, linearen oder verzweigten Kohlenwasserstoffrest mit 1 bis 22 Kohlenstoffatomen und 0, 1 1, 2 oder 3 Doppelbindungen, R² für Wasserstoff oder eine Methylgruppe und n für Zahlen von 1 bis 40 steht, in Gegenwart saurer Katalysatoren mit Carbonsäuren verestert, zur Schaumregulierung in Wasch-, Spül- und Reinigungsmitteln, Lacken und Farben, als Hilfsmittel für die Zucker- und Hefeindustrie sowie in der Abwasserbehandlung von Kläranlagen.

Überraschenderweise wurde gefunden, daß die endgruppenverschlossenen Ester über eine gute schaumdämpfende Wirkung verfügen und auch im alkalischen Milieu über übliche Anwendungszeiträume ausreichend beständig sind. Gegenüber den Mischethern des Stands der Technik ist ihre Herstellung nicht mit dem Zwangsanfall von Salzen verbunden. Zudem zeichnen sie sich durch eine besonders leichte biologische Abbaubarkeit und eine geringe Fischtoxizität aus.

**Fettalkoholpolyglycolether**, die im Sinne der Erfindung als Ausgangsstoffe zur Herstellung der Antischaummittel in Betracht kommen, stellen bekannte Stoffe dar, die großtechnisch durch Anlagerung von Ethylen- und/oder Propylenoxid an primäre Alkohole hergestellt werden. In Abhängigkeit der Natur des verwendeten Katalysators, können die Produkte eine konventionelle oder vorzugsweise auch eine eingeengte Homologenverteilung aufweisen.

Typische Beispiele sind Anlagerungsprodukte von 1 bis 40, vorzugsweise 2 bis 10 Mol Ethylen- und/oder Propylenoxid an Capronalkohol, Caprylalkohol, Caprinalkohol, Laurylalkohol, Myristylalkohol, Cetylalkohol, Palmoleylalkohol, Stearylalkohol, Isostearylalkohol, Oleylalkohol, Elaidylalkohol, Linolylalkohol, Linolenylalkohol, Arachylalkohol, Gadoleylalkohol, Behenylalkohol und Erucylalkohol sowie deren technische Mischungen, wie sie beispielsweise bei der Hydrierung technischer Fettsäuremethylesterfraktionen oder Aldehyde aus der Roelen'schen Oxosynthese anfallen.

Vorzugsweise werden Fettalkoholpolyglycolether der Formel (I) eingesetzt, in der R¹ für Alkylreste mit 2 bis 18, insbesondere 8 bis 18 Kohlenstoffatomen, R² für Wasserstoff und n für Zahlen von 2 bis 10 steht.

Zur Herstellung der Antischaummittel können die genannten Fettalkoholpolyglycolether aliphatischen oder aromatischen, gegebenenfalls hydroxysubstituierten Carbonsäuren mit 6 bis 22 Kohlenstoffatomen verestert werden. Typische Beispiele für aliphatische Carbonsäuren sind Capronsäure, Caprylsäure, Isononansäure, Caprinsäure, Laurinsäure, Myristinsäure, Palmitinsäure, Palmoleinsäure, Stearinsäure, Isostearinsäure, Ölsäure, Elaidinsäure, Linolsäure, Linolensäure, Ricinolsäure, 12-Hydroxystearinsäure, Arachinsäure, Gadoleinsäure, Behensäure und Erucasäure sowie deren technische Mischungen, die man beispielsweise durch Druckspaltung von Fetten und Ölen erhält. Typische Beispiele für aromatische Carbonsäuren sind Benzoesäure und die isomeren Phthalsäuren. Vorzugsweise werden gesättigte aliphatische Carbonsäuren mit 8 bis 18 Kohlenstoffatomen und insbesondere Isononansäure eingesetzt.

Die Veresterung der Polyglycolether mit den Carbonsäuren kann in an sich bekannter Weise erfolgen. Als **saure Katalysatoren** kommen beispielsweise Mineralsäuren, Sulfonsäuren oder saure Ionenaustauscher in Betracht. Typische Beispiele sind Schwefelsäure, Methansulfonsäure, p-Toluolsulfonsäure oder tensidische Sulfosäuren; für die Veresterung aromatischer Carbonsäuren hat sich ferner Zinnschliff als Katalysator bewährt. Es empfiehlt sich, die Katalysatoren in Mengen von 0,5 bis 5 Gew.-% - bezogen auf die Carbonsäuren - einzusetzen.

Die Versterungsreaktion kann bei Temperaturen von 80 bis 120, vorzugsweise 90 bis 110°C durchgeführt werden. Um das Reaktionsgleichgewicht auf die Seite der Produkte zu verlagern, empfiehlt es sich, das Kondensationswasser kontinuierlich zu entfernen. Falls gewünscht, kann der saure Katalysator nach der Veresterung neutralisiert werden.

### Gewerbliche Anwendbarkeit

Die erfindungsgemäß zu verwendenden Antischaummittel zeichnen sich durch eine schaumdämpfende Wirkung und gute ökotoxikologische Verträglichkeit aus.

Die folgenden Beispiele sollen den Gegenstand der Erfindung näher erläutern, ohne ihn darauf einzuschränken.

### Beispiele

### Beispiel 1:

**Herstellung von Octansäure-octanol-4,5 EO-ester**. In einem 500-ml-Dreihalskolben mit Rüher und Destillationsaufsatz wurden 163,1 g (0,5 mol) eines Anlagerungsproduktes von durchschnittlich 4,5 mol Ethylenoxid an Octanol mit eingeengter Homologenverteilung (Dehydol^{(R)} O4-NRE, Fa.Henkel KGaA, Düsseldorf/FRG) und 107,4 g (0,75 mol) Octansäure vorgelegt und mit 1,1 g konz. Schwefelsäure versetzt. Die Mischung wurde auf 110°C erhitzt und das Kondensationswasser unter vermindertem Druck aus dem Gleichgewicht abdestilliert. Nach ca. 160 min wurde der Reaktionsmischung erneut 1,1 g Schwefelsäure zugesetzt und weitere 10 min gerührt. Der Octansäure-4,5 EO-ester wurde in praktisch quantitativer Ausbeute als hellgelbe, leichtbewegliche Flüssigkeit erhalten.

### Beispiel 2:

**Anwendungstechnische Prüfung**. Die entschäumende Wirkung von Octansäure-octanol-4,5 EO-ester wurde in folgender Rezeptur getestet:

| | |
|---|---|
| Triethanolamin | 30 Gew.-% |
| Octansäure | 4 Gew.-% |
| Octansäure-octyl-4,5EO-ester | 4 Gew.-% |
| Wasser, dest. | 62 Gew.-% |

Der Rezeptur wurde als Schaumpromotor 0,25 bis 6 Gew.-% eines Anlagerungsproduktes von durchschnittlich 12 mol Ethylenoxid an ein C_{12/14}-Kokosfettamin zugesetzt. Die Mischung wurde bei einer Temperatur von 31°C über eine Düse in ein Meßgefäß gespritzt, laufend umgepumpt und die sich einstellende Schaumhöhe über der flüsigen Phase abgelesen.

Zum Vergleich wurde die Schaumentwicklung des Kokosamins-12 EO in Wasser (V1) sowie in einer Rezeptur beurteilt, die lediglich 30 Gew.-% Triethanolamin und 4 Gew.-% Octansäure enthielt (V2). Die Ergebnisse sind in Tab.1 zusammengefaßt.

**Tab. 1**

| Schaummessungen in der Spritzapparatur | | |
|---|---|---|
| Bsp. | c(KA) Gew.-% | Schaumhöhe cm |
| 1 | 0,25 | 0 |
| 2 | 0,50 | 0,3 |
| 3 | 1,00 | 0,5 |
| 4 | 2,00 | 1,0 |
| 5 | 3,00 | 1,0 |
| 6 | 4,00 | 1,3 |
| 7 | 5,00 | 1,5 |
| 8 | 6,00 | 2,0 |
| V1 | 6,00 | 12,5 |
| V2 | 6,00 | 11,9 |
| Legende: c(KA) = Konzentration Kokosamin-12EO | | |

## Patentansprüche

1. Verwendung von endgruppenverschlossenen Antischaummitteln, dadurch erhältlich, daß man Fettalkoholpolyglycolether der Formel (I) 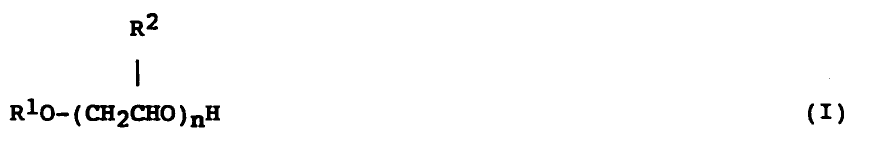 in der R¹ für einen aliphatischen, linearen oder verzweigten Kohlenwasserstoffrest mit 1 bis 22 Kohlenstoffatomen und 0, 1, 2 oder 3 Doppelbindungen, R² für Wasserstoff oder eine Methylgruppe und n für Zahlen von 1 bis 40 steht, in Gegenwart saurer Katalysatoren mit Carbonsäuren verestert, zur Schaumregulierung in Wasch-, Spül- und Reinigungsmitteln, Lacken und Farben, als Hilfsmittel für die Zucker- und Hefeindustrie sowie in der Abwasserbehandlung von Kläranlagen.

2. Verwendung nach Anspruch 1, **dadurch gekennzeichnet**, daß man Fettalkoholpolyglycolether der Formel (I) einsetzt, in der R¹ für Alkylreste mit 2 bis 18 Kohlenstoffatomen, R² für Wasserstoff und n für Zahlen von 2 bis 10 steht.

3. Verwendung nach den Ansprüchen 1 und 2, **dadurch gekennzeichnet**, daß man aliphatische oder aromatische, gegebenenfalls hydroxysubstituierte Carbonsäuren mit 6 bis 22 Kohlenstoffatomen einsetzt.

4. Verwendung nach den Ansprüchen 1 bis 3, **dadurch gekennzeichnet**, daß man als saure Katalysatoren Mineralsäuren, Sulfonsäuren oder saure Ionenaustauscher einsetzt.

## Claims

1. The use of end-capped antifoam agents obtainable by esterification of fatty alcohol polyglycol ethers corresponding to formula (I): 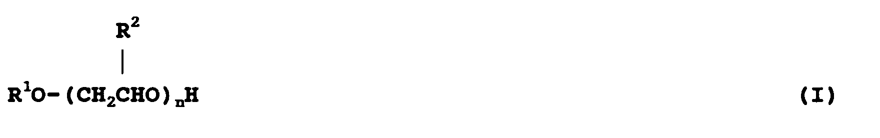 in which R¹ is an aliphatic, linear or branched hydrocarbon radical containing 1 to 22 carbon atoms and 0, 1, 2 or 3 double bonds, R² is hydrogen or a methyl group and n is a number of 1 to 40,
with carboxylic acids in the presence of acidic catalysts,
for foam control in laundry detergents, dishwashing detergents and cleaning products, paints and lacquers, as auxiliaries for the sugar and yeast industry and in the wastewater treatment of sewage plants.

2. The use claimed in claim 1, **characterized in that** fatty alcohol polyglycol ethers corresponding to formula **(I)**, in which R¹ represents C₂₋₁₈ alkyl radicals, R² is hydrogen and n is a number of 2 to 10, are used.

3. The use claimed in claims 1 and 2, **characterized in that** aliphatic or aromatic, optionally hydroxy-substituted carboxylic acids containing 6 to 22 carbon atoms are used.

4. The use claimed in claims 1 to 3, **characterized in that** mineral acids, sulfonic acids or acidic ion exchangers are used as the acidic catalysts.

## Revendications

1. Utilisation d'agents antimousse fermés au niveau du groupe terminal, obtenables par estérification de polyglycoléthers d'alcools gras de la formule (I) 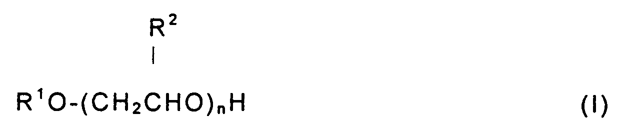 dans laquelle R¹ représente un radical d'hydrocarbure aliphatique, linéaire ou ramifié, comportant 1 à 22 atomes de carbone et 0, 1, 2 ou 3 doubles liaisons, R² correspond à l'hydrogène ou à un groupe méthyle et n est un nombre de 1 à 40, avec des acides carboxyliques, en présence de catalyseurs acides, afin de réguler la mousse dans les agents de lavage, de rinçage et de nettoyage, les laques et les peintures, comme adjuvant pour l'industrie du sucre et de la levure, ainsi que dans le traitement des eaux usées des stations d'épuration.

2. Utilisation selon la revendication 1, **caractérisée en ce que** l'on met en oeuvre des polylglycoléthers d'alcools gras de la formule (I), dans laquelle R¹ représente un radical alkyle comportant 2 à 18 atomes de carbone, R² correspond à l'hydrogène et n est un nombre de 2 à 10.

3. Utilisation selon les revendications 1 et 2, **caractérisée en ce que** l'on met en oeuvre des acides carboxyliques aliphatiques ou aromatiques, éventuellement à substitution hydroxyle, comportant 6 à 12 atomes de carbone.

4. Utilisation selon les revendications 1 à 3, **caractérisée en ce que** l'on met en oeuvre comme catalyseurs acides, des acides minéraux, des acides sulfoniques ou des échangeurs d'ions acides.
